(19) Europäisches Patentamt
European Patent Office
Office européen des brevets

(11) Veröffentlichungsnummer: **0 374 647 B1**

(12) # EUROPÄISCHE PATENTSCHRIFT

(45) Veröffentlichungstag der Patentschrift: **15.06.94**

(21) Anmeldenummer: **89122749.8**

(22) Anmeldetag: **09.12.89**

(51) Int. Cl.5: **C07C 213/02**, C07C 217/40, C07D 317/28, C07D 319/04, C07C 217/64

(54) **Verfahren zur Herstellung von optisch aktiven alpha-Aminoacetalen.**

(30) Priorität: **23.12.88 DE 3843390**

(43) Veröffentlichungstag der Anmeldung:
**27.06.90 Patentblatt 90/26**

(45) Bekanntmachung des Hinweises auf die Patenterteilung:
**15.06.94 Patentblatt 94/24**

(84) Benannte Vertragsstaaten:
**CH DE ES FR GB IT LI NL**

(56) Entgegenhaltungen:

**TETRAHEDRON, Band 30, 1974, Seiten 4233-4237, Oxford, GB; M. GACEK et al.: "N-Ouaternary compounds-IXL; Circular dichroism of alpha-trimethylammonio aldehydes"**

**SYNTHESIS, Nr. 8, August 1989, Seiten 608-610, Stuttgart, DE; G. BRINGMANN etal.: "A simple, chiral-pool-independent synthesis of enantiomerically pure alanine-derived alpha-amino aldehyde acetals"**

**Chem. Ber. 117, 1984, 2076-2099**

**Angew. Chem. 98, 1986, 917-919**

**J. Med. Chem. 16, 1973, 480-483**

**J. Med. Chem. 19, 1976, 1400-1404**

**Morrison/Mosher: "Asymmetric Organic Reactions", Prentice Hall, Inc., Englewood Cliffs, New Jersey, 1971, Seiten 303-321**

(73) Patentinhaber: **BASF Aktiengesellschaft**
**Carl-Bosch-Strasse 38**
**D-67063 Ludwigshafen(DE)**

(72) Erfinder: **Geisler, Jörg-Peter**
**Horstmarer Landweg**
**D-8700 Würzburg(DE)**
Erfinder: **Bringmann, Gerhard, Prof.Dr.**
**Gertrud-von-le-Fort-Strasse 412**
**D-8700 Würzburg(DE)**
Erfinder: **Jansen, Johannes Rudolf**
**Benteler Strasse 35**
**D-4400 Münster(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft ein neues Verfahren zur Herstellung von optisch aktiven α-Aminoacetalen der allgemeinen Formel I

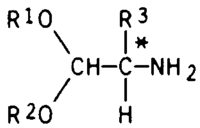

I

* Chiralitätszentrum

in der $R^1$ und $R^2$ für $C_1$-$C_4$-Alkylreste stehen, die auch zu einem 5- oder 6-gliedrigen Ring verbunden sein können, und $R^3$ einen $C_1$-$C_{24}$-Alkylrest oder einen Arylrest bedeutet.

Bisher bekannte Synthesen für α-Aminoacetale gehen von den natürlichen α-Aminosäuren oder den daraus erhältlichen entsprechenden α-Aminoalkoholen aus.

Nach der Lehre der DE-A 37 11 911 kann man α-Aminosäureester, deren Aminogruppe geschützt ist, mit Diisobutylaluminiumhydrid zu den entsprechenden Aldehyden reduzieren, oder man kann α-Aminoalkohole nach Swern mit aktiviertem Dimethylsulfoxid zu den α-Aminoaldehyden oxidieren.

Die Oxidation von α-Aminoalkoholen mit aktiviertem Dimethylsulfoxid und die anschließende Acetalisierung wird auch von S. Kano et al., Heterocycles 26, 2805 (1987), beschrieben.

Es ist auch bekannt, durch Reduktion der Säurechloride von α-Aminosäuren zu α-Aminoaldehyden zu gelangen, die dann acetalisiert werden (K. Balenkovic et al., J. Org. Chem., 18, 297 (1953); M. Gacek und K. Undheim, Tetrahedron 30, 4233 (1974)).

Nachteilig an all diesen Verfahren ist jedoch, daß die Stufe des konfigurationslabilen Aldehyds durchlaufen wird, was eine teilweise Racemisierung und damit geringere Enantiomerenreinheit der Endprodukte zur Folge hat. Außerdem sind diese Verfahren nur an N-geschützten Verbindungen durchführbar, so daß man die Schutzgruppen zusätzlich wieder von der Aminofunktion abspalten muß.

Es ist ferner bekannt, Ketone, die an einem oder beiden α-C-Atomen mit Kohlenwasserstoffresten substituiert sind, die ihrerseits z.T. an entfernteren Molekülteilen inerte Substituenten wie Methoxygruppen tragen, mit Hilfe eines optisch aktiven Amins gemaß folgendem Schema

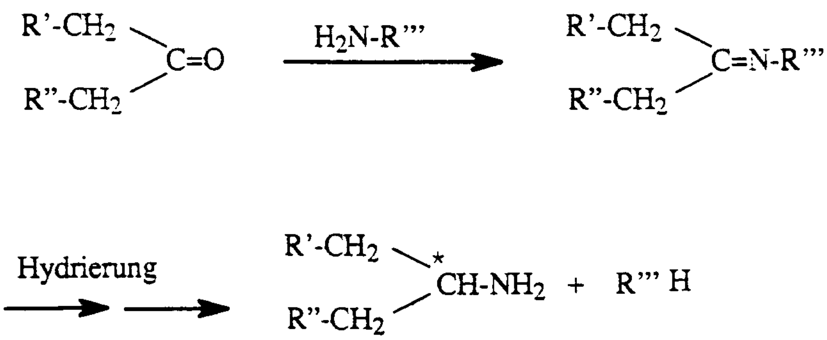


R' ≠ R'' Kohlenwasserstoffreste

R''' Rest eines optisch aktiven Amins

in optisch aktive Amine hoher optischer Ausheute zu überführen (Chem. Ber. 117, 2076 (1984); Angew. Chem. 98, 917 (1986); J. Med. Chem. 16, 480 (1973); ibid. 19, 1400 (1976)).

Eine ähnliche Reaktion zur Herstellung von optisch aktiven α-Aminosäuren aus α-Ketocarbonsäuren gelingt jedoch bezüglich der optischen Ausbeuten sehr schlecht (vgl. J.D. Morrison/H.S. Mosher "Asymmetric Organic Reactions", Prentice Hall, Inc., Englewood Cliffs, New Jersey, 1971, Seiten 303 - 321), was vermutlich auf den stark aktivierenden Einfluß der Carboxylgruppe in Nachbarstellung zur Carbonylgruppe zurückzuführen ist. Allgemein ist hieraus zu schließen, daß im Falle aktivierender Substituenten an der

Carbonylgruppe bei dem in Rede stehenden Reaktionsprinzip nur unbefriedigende Ergebnisse zu erwarten sind.

Ein weiterer Nachteil dieser Synthesen besteht darin, daß die natürlichen Aminosäuren überwiegend nur in der L-Konfiguration zur Verfügung stehen.

Aufgabe der Erfindung war es daher, diesen Mängeln abzuhelfen.

Demgemäß wurde ein neues Verfahren zur Herstellung von optisch aktiven α-Aminoacetalen der allgemeinen Formel I

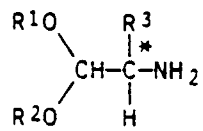

I

* Chiralitätszentrum

in der $R^1$ und $R^2$ für $C_1$-$C_4$-Alkylreste stehen, die auch zu einem 5- oder 6-gliedrigen Ring verbunden sein können, und $R^3$ einen $C_1$-$C_{24}$-Alkylrest oder einen Arylrest bedeutet, gefunden, welches dadurch gekennzeichnet ist, daß man α-Oxoacetale der allgemeinen Formel II

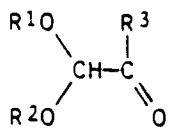

II

mit einem optisch aktiven 1-Phenyl-ethylamin III zu der Schiff'schen Base IV

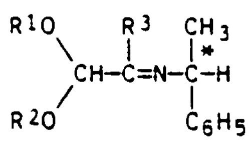

IV

umsetzt, diese zu dem Aminoacetal V

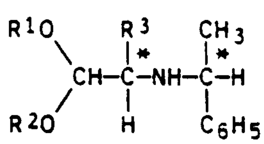

V

hydriert, und V anschließend einer hydrierenden Spaltung unterwirft.

Das erfindungsgemäße Verfahren geht von den prochiralen α-OxoacetalenII aus, die in an sich bekannter Weise leicht herstellbar sind. So kann man beispielsweise 1,1-Dimethoxy-2-oxo-propan nach A. Wohl und M. Lange, Chem. Ber. 43, 3665 (1908) durch Umsetzung von Dialkoxyessigsäureamiden mit Methylmagnesiumjodid und anschließender Abspaltung eines Amins darstellen.

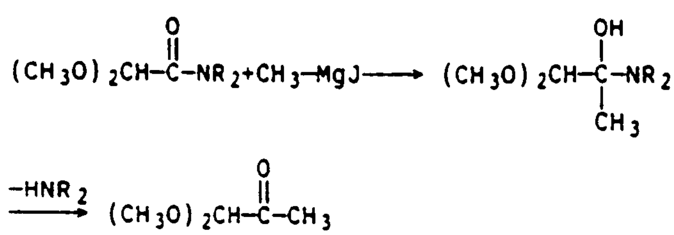


Die α-Oxoacetale II werden mit dem optisch aktiven 1-Phenylethylamin III umgesetzt, wobei wahlweise das R- oder S-Enantiomere von III eingesetzt werden kann. 1-Phenylethylamin ist in seiner optisch aktiven Form

leicht durch Racematspaltung mit Mandelsäure zugänglich. Im allgemeinen hat das eingesetzte 1-Phenylethylamin eine Enantiomerenreinheit von ca. 95 %.

Die Umsetzung von II mit III zu den Diastereomeren V erfolgt in an sich bekannter Weise:
Zunächst werden durch Umsetzung von II mit einem optisch aktiven 1-Phenylethylamin III die Schiff'schen Basen IV gebildet (Stufe A), woran sich eine Hydrierung zu den diastereomeren Verbindungen V anschließt (Stufe B).

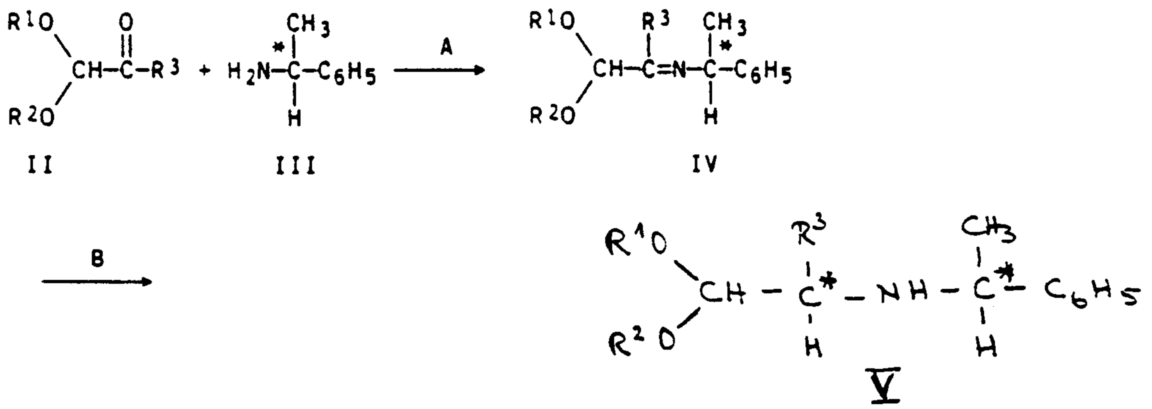


II III IV

V

Der Reaktionsschritt A kann unter den für solche Kondensationen üblichen Bedingen durchgeführt werden, beispielsweise in dem man II und III in einer toluolischen Lösung unter kontinuierlicher destillativer Entfernung des Reaktionswassers miteinander umsetzt, wobei zusätzlich katalytische Mengen an p-Toluolsulfonsäure eingesetzt werden können. Die beiden Komponenten II und III werden im allgemeinen im equimolaren Verhältnis eingesetzt.

Die entstehende Schiff'sche Base enthält das Chiralitätszentrum des Hilfsreagens III in unveränderter Form.

Die Hydrierung der Schiff'schen Base IV kann unter relativ milden Bedingungen erfolgen, z.B. mit Raney-Nickel bei 20-80 °C und 1 - 5 bar.

Durch die asymmetrische Induktion des bereits vorhandenen Chiralitätszentrums in IV wird das bei der Hydrierung entstehende zweite Chiralitätszentrum in V in hoher Stereoselektivität erhalten.

Es ist nicht nötig, die Schiff'schen Basen IV für die Hydrierung zu isolieren, vielmehr kann man die Hydrierung (B) direkt im Anschluß an die Kondensation (A) vornehmen.

Falls das so hergestellte Diastereomer V nicht in ausreichender optischer Reinheit erhalten wird, kann es durch Säulenchromatographie oder durch Umkristallisation geeigneter Derivate, beispielsweise als Salz der Perchlorsäure, gereinigt werden.

In einer sich anschließenden Stufe (C) wird der Phenylethylrest aus V durch hydrierende Spaltung entfernt, so daß man die $\alpha$-Aminoacetale I erhält.

Die hydrierende Spaltung wird vorzugsweise als Transferhydrogenolyse vorgenommen, beispielsweise mit Ammoniumformiat als $H_2$-Quelle und Palladium als Katalysator. Das Palladium wird zweckmäßigerweise in Form handelsüblicher Trägerkatalysatoren aus Aktivkohle mit Pd-Gehalten von 2-20, vorzugsweise 5-15 Gew.-%, eingesetzt.

Als Lösungsmittel eignen sich polare, protische Flüssigkeiten wie Wasser oder niedere Alkohole, z.B. Methanol.

Es ist aber auch möglich, die hydrierende Spaltung der Verbindungen V mit Wasserstoff unter erhöhtem Druck von 1-200 bar und bei 20-50 °C vorzunehmen, wobei als Hydrierkatalysator ebenfalls ein Palladium-Katalysator, wie für die Transferhydrogenolyse beschrieben, eingesetzt werden kann. Als Lösungsmittel eignen sich diejenigen, die für die Transferhydrogenolyse genannt wurden.

Im übrigen ist die hydrogenolytische Spaltung von Aminen eine allgemein bekannte Methode, die keine verfahrenstechnischen Besonderheiten aufweist.

Die Aufarbeitung auf die Verfahrensprodukte I erfolgt unter üblichen Bedingungen, etwa indem man die I enthaltende Phase in Alkalilauge, z.B. NaOH, aufnimmt und mit einen wasserunlöslichen Solvens extrahiert. Zur Extraktion geeignete Solventien sind beispielsweise Ether wie Diethylether.

Danach ist eine destillative Reinigung der Produkte möglich.

Die Überprüfung der Enantiomerenreinheit kann nach der Methode von Mosher et al. vorgenommen werden (vgl. J.A. Dale, H.S. Mosher, J.Am.Chem.Soc.95, 512 (1975)).

Das erfindungsgemäße Verfahren eignet sich beispielsweise zur Herstellung von α-Aminoacetalen I in denen $R^1$, $R^2$ und $R^3$ folgende Bedeutung haben:

$R^1$ u. $R^2$: Methyl-, Ethyl-, n-Propyl, Isopropyl-, n-Butyl oder Ethylen-, Propylen-, wenn $R^1$ und $R^2$ zu einem 5- oder 6-gliedrigen Ring verknüpft sind

$R^3$: Methyl-, Ethyl-, Isopropyl-, n,s-Butyl-, Phenyl-, 3-Methoxyphenyl-, 2,4-Dimethoxyphenyl-, 2,3,4-Trimethoxyphenyl

Die nach dem erfindungsgemäßen Verfahren erhältlichen optisch aktiven α-Aminoacetale I sind interessante bifunktionelle und chirale Bausteine für Naturstoffsynthesen.

Beispiel 1

Herstellung von S-1,1-Dimethoxy-2-propanamin

I) Eine Lösung aus 20.0 g (0.165 mol) S-1-Phenylethylamin und 19.5 g (0.165 mol) 1,1-Dimethoxy-propan-2-on und 300 ml Toluol wurde 5 Stunden am Wasserabscheider erhitzt. Anschließend wurde das Lösungsmittel unter vermindertem Druck verdampft, wonach der ölige Rückstand mit 200 ml wasserfreiem Ethanol aufgenommen wurde. Diese Lösung wurde rasch in eine Hydrierapparatur überführt und mit 8,0 g ethanolfeuchtem Raney-Nickel versetzt. Anschließend wurde bei einem Wasserstoffdruck von 5 bar und 25 °C 24 Stunden lang hydriert. Nach Abfiltrieren des Katalysator und Einengen des Rückstandes unter vermindertem Druck auf ca. 50 ml wurden durch Zugabe von 25 ml 70 gew.-%iger Perchlorsäure und weiteres Einengen die sekundären Amine als Hydroperchlorate gefällt. Diese wurden aus Dichlormethan/Petrolether umkristallisiert. Man erhielt diastereomerenreines (2 S, 1's)-N-(1'-Phenylethyl)-1,1-dimethoxy-2-propanamin ("de" = 100 %) mit einer Ausbeute von 44,9 g (0,14 mol; 85 %).
Schmp.: 108 °C
$[\alpha]_{D'}^{20}$ = -13,9 °
II) 10,0 g (0,031 mol) des nach I erhaltenen Diastereomeren wurden unter Stickstoffatmosphäre in 100 ml absolutem Methanol gelöst und mit 6,8 g (0,108 mol) Ammoniumformiat sowie 200 mg eines Palladium/Aktivkohle-Trägerkatalysators (10 Gew.-% Pd) versetzt und solange unter Rückfluß erhitzt, bis nach ca. 45 min dünnschichtchromatographisch das Ende der Reaktion angezeigt wurde. Nach der üblichen Aufarbeitung wurde der Rückstand im Wasserstrahlvakuum destilliert.

Man erhielt 3,5 g (0,029 mol) an S-1,1-Dimethoxy-2-propanamin als farblose, schwach viskose Flüssigkeit, was einer Ausbeute von 95 % entspricht.
Sdp.: 45 °C (25 mm Hg)
$\alpha]_{D'}^{20}$ = + 3,7 °
"ee" = > 98 %

Beispiel 2

Herstellung von R-1,1-Dimethoxy-2-propanamin

Die Herstellung des R-Enantiomeren erfolgte analog Bsp. 1.
(2R, 1'R)-N-(1'-Phenylethyl)-1,1-dimethoxy-2-propanamin wurde mit einer
I) Ausbeute von 80 % (42,7 g; 0,132 mol) erhalten.
Schmp.: 107 °C
$[\alpha]_{D'}^{20}$ = + 13,9 °
"de" = > 98 %
II) Das R-1,1-Dimethoxy-2-propanamin wurde mit einer Ausbeute von 82 % erhalten.
Sdp.: 45 °C (25 mm Hg)
$[\alpha]_{D'}^{20}$ = -4,0 °
"ee" = 98 %

## Patentansprüche

**1.** Verfahren zur Herstellung von optisch aktiven α-Aminoacetalen der allgemeinen Formel I

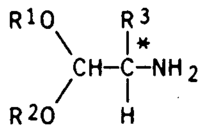

I

* Chiralitätszentrum

in der $R^1$ und $R^2$ für $C_1$-$C_4$-Alkylreste stehen, die auch zu einem 5- oder 6-gliedrigen Ring verbunden sein können, und $R^3$ einen $C_1$-$C_{24}$-Alkylrest oder einen Arylrest bedeutet, dadurch gekennzeichnet, daß man α-oxoacetale der allgemeinen Formel II

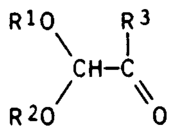

II

mit einem optisch aktiven 1-Phenyl-ethylamin III zu der Schiff'schen Base IV

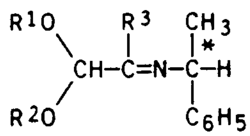

IV

umsetzt, diese zu dem Aminoacetal V

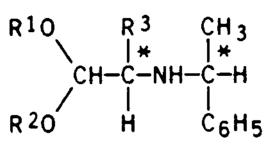

V

hydriert, und V anschließend einer hydrierenden Spaltung unterwirft.

**2.** Verfahren nach Anspruch 1, dadurch gekennzeichnet, daß man die hydrierende Spaltung von V als Transferhydrogenolyse mit Ammoniumformiat und einem Edelmetall der VIII. Nebengruppe als Hydrierkatalysator durchführt.

## Claims

**1.** A process for preparing optically active α-amino acetals of the formula I

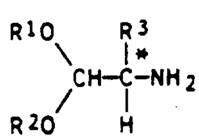

I

* chirality center

where $R^1$ and $R^2$ are $C_1$-$C_4$-alkyl radicals which can also be linked to form a 5- or 6-membered ring, and $R^3$ is a $C_1$-$C_{24}$-alkyl radical or an aryl radical, which comprises reacting $\alpha$-oxo acetals of the formula II

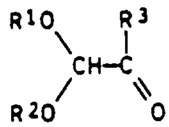

II

with an optically active 1-phenylethylamine III to give the Schiff's base IV

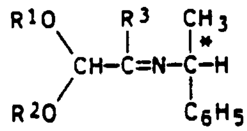

IV

hydrogenating the latter to give the amino acetal V

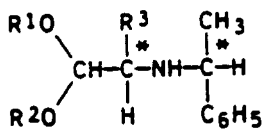

V

and subsequently subjecting V to hydrogenating cleavage.

2. A process as claimed in claim 1, wherein the hydrogenating cleavage of V is carried out as transfer hydrogenolysis with ammonium formate and a noble metal of group VIII as hydrogenation catalyst.

## Revendications

1. Procédé de préparation de $\alpha$-aminoacétales optiquement actifs de la formule générale I

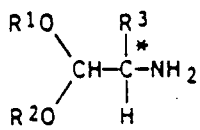

I

* Centre de chiralité

dans laquelle $R^1$ et $R^2$ sont mis pour des restes alkyle en $C_1$-$C_4$ qui peuvent aussi être liés pour former un noyau à 5 ou 6 chaînons, et $R^3$ signifie un reste alkyle en $C_1$-$C_{24}$ ou un reste aryle, caractérisé par le fait que l'on transforme des $\alpha$-oxoacétales de la formule générale II

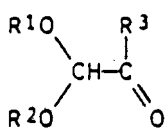

II

avec une phényl-éthylamine III optiquement active, en la base de Schiff IV

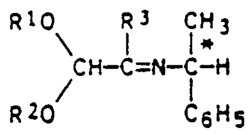

IV

on hydrogène celle-ci en l'aminoacétal V

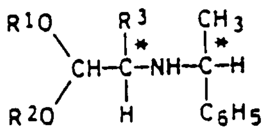

V

et on soumet ensuite V à une scission hydrogénante.

2. Procédé selon la revendication 1, caractérisé par le fait que l'on effectue la scission hydrogénante de V, comme hydrogénolyse de transfert avec du formiate d'ammonium et un métal noble du groupe secondaire VII comme catalyseur d'hydrogénation.